(19) Europäisches Patentamt
European Patent Office
Office européen des brevets

(11) Publication number: **0 044 504**
**A1**

(12) **EUROPEAN PATENT APPLICATION**

(21) Application number: 81105485.7

(22) Date of filing: 13.07.81

(51) Int. Cl.$^{3}$: C 07 D 333/38

(30) Priority: 22.07.80 IT 2360080

(43) Date of publication of application: 27.01.82 Bulletin 82/4

(84) Designated Contracting States: AT BE CH DE FR GB IT LI NL

(71) Applicant: NEOPHARMED S.p.A.
Via Pordoi, 18
I-20021 Baranzate di Bollate Milano(IT)

(72) Inventor: Reiner, Alberto
Via Mentana 23
Como(IT)

(74) Representative: Bühling, Gerhard, Dipl.-Chem. et al,
Patentanwaltsbüro
Tiedtke-Bühling-Kinne-Grupe-Pellmann-Dragotti
Bavariaring 4
D-8000 München 2(DE)

(54) **A derivative of theonyl thio-propionyl-glycine and process for the preparation thereof.**

(57) The salt of (DL)-lysine of the theonyl-thio-propionyl-glycine is not hygroscopic, whereby both the handling and the long term unalterability of the active substance are permitted.

EP 0 044 504 A1

"A derivative of theonyl thio-propionyl-glycine and process for the preparation thereof"

oooooooooo

The present invention relates to a stable derivative of 2-(α-theonyl-thio)-thio-propionyl-glycine, (hereinafter shortly indicated by theonyl-thio-propionyl-glycine), namely the salt of (DL)-lysine, having the formula:

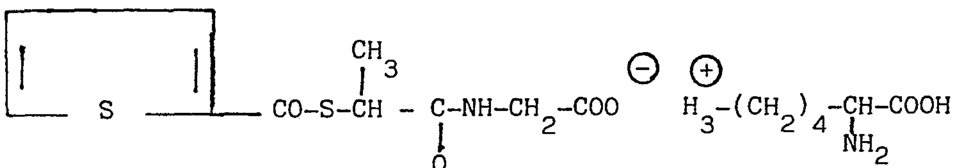

The theonyl-thio-propionyl-glycine, as the soluble sodium salt, is endowed with optimum expectorant properties and thus with interesting therapeutical prospects. However, it is a highly hygroscopic compound, the chemical isolation of which is particularly difficult; even the lyophilization treatment gives place to a little stable product, which is readily degradable mainly due to the high hygroscopicity.

There are thus evident the problems raised by this compound under the point of view of the industrial handling and of the use.

It has been now found that the salt of theonyl-thio-propionyl-glycine with (DL)-lysine is not hygroscopic, whereby the above mentioned problem is wholly solved without interferring with or affecting the therapeutical properties of the basic active principle.

It is a compound having melting point of 173-175°C, which is a white, microcrystalline powder, completely soluble in water, whereby no problems exist as regards the preparation of the several pharmaceutical forms.

For the preparation of the compound of the invention, a process is foreseen according to which theonyl-thio-propionyl-glycine and (DL)-lysine are reacted in stechiometric ratio, in alcoholic solvent and at a temperature of 20-50°C.

The following examples illustrate the invention without limiting purpose.

EXAMPLE 1

5.4 g of theonyl-thio-propionyl-glycine (0.02 moles) are charged in a becker there

being orderly added 40 mls of isopropyl alcohol and 5.6 g of 50% (DL)-lysine base (0.02 moles).

The temperature of the reaction mixture is brought to 40°C and the reaction takes place with conversion of the mass without a complete phase and with precipitation of the reaction product.

The reaction mixture is very slowly filtered, due to the presence of swollen crystals and dried in an oven.

7 g of product are obtained, having melting point of 173-185°C.

EXAMPLE 2

The process of example 1 is repeated, except that the reaction solvent is in this case ethanol (50 mls), it being added to the reaction mixture in two steps, namely 10 mls at the beginning and the remaining 40 mls after the lysine addition.

In this case the reaction product crystallizes and there are obtained 6 g (dry) of crystalline product, which under thin layer chromatography appears as a pure compound.

EXAMPLE 3

The process of example 1 is repeated, except that methanol (50 mls) is used as the reaction solvent.

In this case the product does not crystallize and for the crystallization 20 mls of isopropyl alcohol must be added.

CLAIMS

1) (DL)-lysine salt of 2-(α-theonylthio)-thiopropionyl-glycine, having the formula:

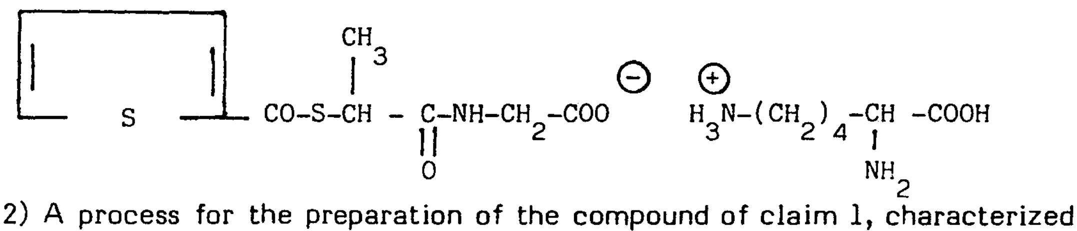

2) A process for the preparation of the compound of claim 1, characterized in that theonyl-thio-propionyl-glycine and (DL)-lysine base are reacted in stechiometrical ratio, in an alcholic solvent and at a temperature of 20 to 50°C.

3) A process according to claim 2,characterized in that said alcoholic solvent is selected among methanol, ethanol and isopropanol.

0044504

European Patent Office

**EUROPEAN SEARCH REPORT**

Application number
EP 81 10 5485

| DOCUMENTS CONSIDERED TO BE RELEVANT | | | CLASSIFICATION OF THE APPLICATION (Int Cl.³) |
|---|---|---|---|
| Category | Citation of document with indication, where appropriate, of relevant passages | Relevant to claim | |
| | GB - A - 2 018 756 (MEDIOLANUM FARM)<br>* Claim 1 *<br>---- | 1 | C 07 D 333/38 |
| | | | TECHNICAL FIELDS SEARCHED (Int. Cl.³) |
| | | | C 07 D 333/38 |
| | | | CATEGORY OF CITED DOCUMENTS<br>X: particularly relevant<br>A: technological background<br>O: non-written disclosure<br>P: intermediate document<br>T: theory or principle underlying the invention<br>E: conflicting application<br>D: document cited in the application<br>L: citation for other reasons<br>&: member of the same patent family corresponding document |
| ✓ | The present search report has been drawn up for all claims | | |

| Place of search | Date of completion of the search | Examiner |
|---|---|---|
| The Hague | 14-10-1981 | CHOULY |